(19) Europäisches Patentamt
European Patent Office
Office européen des brevets

(11) EP 1 260 502 A1

(12) **EUROPEAN PATENT APPLICATION**
published in accordance with Art. 158(3) EPC

(43) Date of publication:
**27.11.2002 Bulletin 2002/48**

(21) Application number: **01906339.5**

(22) Date of filing: **27.02.2001**

(51) Int Cl.[7]: **C07C 401/00**
// (A61K31/59, A61P3:02)

(86) International application number:
**PCT/JP01/01451**

(87) International publication number:
**WO 01/062723 (30.08.2001 Gazette 2001/35)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **28.02.2000 JP 2000050915**

(71) Applicant: **CHUGAI SEIYAKU KABUSHIKI KAISHA**
**Tokyo, 115-8543 (JP)**

(72) Inventors:
- **TAKAYAMA, Hiroaki**
**Shibuya-ku, Tokyo 151-0072 (JP)**
- **KITTAKA, Atsushi**
**Kunitachi-shi, Tokyo 186-0002 (JP)**
- **SUHARA, Yoshitomo**
**Yokohama-shi, Kanagawa 226-0025 (JP)**
- **FUJISHIMA, Toshie**
**Hachioji-shi, Tokyo 193-0834 (JP)**

(74) Representative: **VOSSIUS & PARTNER**
**Siebertstrasse 4**
**81675 München (DE)**

(54) **VITAMIN D DERIVATIVES HAVING SUBSTITUENTS AT THE 2ALPHA-POSITION**

(57) An object of the present invention is to synthesize a novel vitamin D derivative having a substituent at the 2α-position.

According to the present-invention, there is provided a vitamin D derivative of Formula (1):

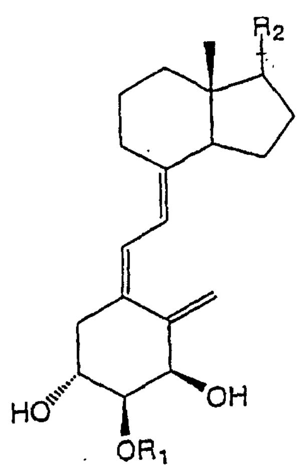

wherein $R_1$ and $R_2$ are straight chained or branched lower alkyl which may be substituted with hydroxy.

EP 1 260 502 A1

## Description

### TECHNICAL FIELD

The present invention relates to novel vitamin D derivatives, more particularly, relates to vitamin D derivatives having a substituent at the 2α-position.

### BACKGROUND ART

Vitamin D derivatives are known to exhibit a variety of physiological activities such as calcium metabolism regulatory activities, growth-inhibitory and differentiation-inducing activities for tumor cells, and immunoregulatory activities, and various vitamin D derivatives are proposed as therapeutic agents for nephrogenic bone diseases, hypoparathyroidism, osteoporosis and the like.

Among numerous vitamin D derivatives, those which have a substituent at the 2β-position possess physiological activities such as in vivo calcium metabolism-controlling activity and differentiation-inducing activity on cells such as tumor cells and are reported to be useful as medicines, such as therapeutic agents for diseases associated with abnormal calcium metabolism, for example, osteoporosis and osteomalacia, and antitumor agents (Japanese Patent Publication (Kokoku) No. 6-23185).

As 2α-substituted vitamin D derivatives, those having 4-hydroxybutyl or acyloxy at the 2α-position were known (J. Org. Chem., Vol.59, No.25, 1994 and Japanese Patent Publication (Kokai) No. 51-19752). However, the number of reports of 2α-substituted vitamin D derivatives is less than that of 2β-substituted vitamin D derivatives, and development of 2α-substituted vitamin D derivatives having higher physiological activities has been desired.

Thus, paying attention to 2α-substituted vitamin D derivatives, the inventors of the present invention conducted various studies on their physiological activities and other characteristics.

### DISCLOSURE OF THE INVENTION

An object of the present invention is to synthesize and provide a novel vitamin D derivative, in which a specific substituent is introduced to the 2α-position and the hydroxy at 1- and 3-positions are α- and β-oriented, respectively.

Another object of the present invention is to provide a novel vitamin D derivative possessing excellent binding property to vitamin D receptor.

As a result of careful studies as to achieve the above mentioned objects, the inventors of the present invention have found that the binding property to vitamin D receptor is increased by introducing a specific substituent to the 2α-position and thereby achieved the present invention.

Accordingly, the present invention provides a vitamin D derivative of Formula (1):

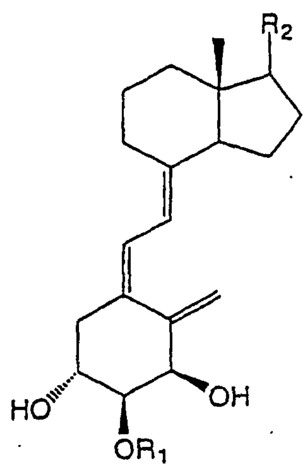

wherein $R_1$ and $R_2$ are straight chained or branched lower alkyl which may be substituted with hydroxy.

Preferably, $R_1$ is straight chained $C_{2-6}$ alkyl substituted with hydroxy and $R_2$ is straight chained or branched lower $C_{1-12}$ alkyl substituted with hydroxy in Formula (1).

More preferably, $R_1$ is straight chained $C_{2-4}$ alkyl substituted with hydroxy and $R_2$ is straight chained or branched lower $C_{3-10}$ alkyl substituted with hydroxy.

According to another aspect of the present invention, there is provided a vitamin D derivative of Formula (2):

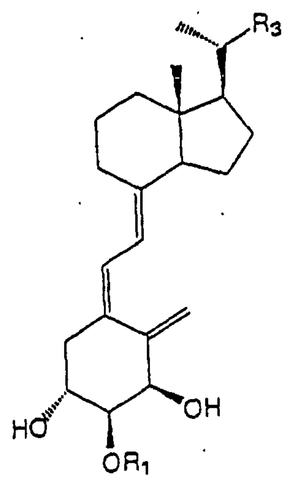

wherein $R_1$ is 2-hydroxyethyl or 3-hydroxypropyl and $R_3$ is 4-hydroxy-4-methylpentyl or 4-ethyl-4-hydroxyhexyl.

Preferably, $R_1$ is 3-hydroxypropyl and $R_3$ is 4-hydroxy-4-methylpentyl in Formula (2).

According to another aspect of the present invention, there is provided use of the vitamin D derivative of the present invention for the manufacture of a medicament for treating diseases accompanied by abnormal calcium metabolism.

According to another aspect of the present invention, there is provided a method of treating diseases accompanied by abnormal calcium metabolism, comprising the step of administering to a host in need of such a treatment a therapeutically effective amount of the vitamin D derivative of the present invention.

The vitamin D derivative of the present invention may be useful as a reagent for studying metabolism of active vitamin $D_3$ (i.e., 1$\alpha$,25-dihydroxyvitamin $D_3$).

The whole content of Japanese Patent Application No. 2000-050915, on which the claim for the priority of the present application is based, is incorporated herein by reference.

## PREFERRED MODE FOR CARRYING OUT THE INVENTION

In Formula (1), $R_1$ and $R_2$ represent straight chained or branched lower alkyl which may be substituted with hydroxy.

In the present specification, straight chained or branched lower alkyl generally means straight chained or branched $C_{1-15}$ alkyl; examples thereof include methyl, ethyl, n-propyl, i-propyl, n-butyl, s-butyl, i-butyl and t-butyl, and also include pentyl, hexyl, heptyl, octyl, nonyl, decanyl, etc.

Straight chained or branched lower alkyl which may be substituted with hydroxy means that any hydrogen atom of the above-mentioned lower alkyl may be substituted with one or more hydroxy.

The number of hydroxy substituents each of $R_1$ and $R_2$ may have is 1, 2 or 3, preferably 1 or 2, and more preferably 1.

$R_1$ is preferably straight chained $C_{2-6}$ alkyl substituted with hydroxy and more preferably straight chained $C_{2-4}$ alkyl substituted with hydroxy. Non-limiting examples of $R_1$ include hydroxymethyl, hydroxyethyl, hydroxypropyl, hydroxybutyl, hydroxypentyl, hydroxyhexyl, etc.

$R_2$ is preferably straight chained or branched lower $C_{1-12}$ alkyl substituted with hydroxy and more preferably straight chained or branched lower $C_{3-10}$ alkyl substituted with hydroxy. Non-limiting examples of $R_2$ include 6-hydroxy-6-methyl-2-heptyl, 7-hydroxy-7-methyl-2-octyl, 5,6-dihydroxy-6-methyl-2-heptyl, 4,6,7-trihydroxy-6-methyl-2-heptyl, etc.

The vitamin D derivatives of the present invention are preferably compounds of Formula (2). Preferably, $R_1$ is 2-hydroxyethyl or 3-hydroxypropyl, more preferably 3-hydroxypropyl in Formula (2). Preferably, $R_3$ is 4-hydroxy-4-methylpentyl or 4-ethyl-4-hydroxyhexyl, more preferably 4-hydroxy-4-methylpentyl.

Among compounds of Formula (1) of the present invention, (5Z,7E)-(1S,2S,3R,20R)-2-(3-hydroxypropoxy)-9,10-seco-5,7,10(19)-cholestatriene-1,3,25-triol is preferred.

The vitamin D derivatives of the present invention can be used as medicaments, for example, as therapeutic agents for diseases accompanied by abnormal calcium metabolism, anti-tumor agents, immunomodulators and the like.

Furthermore, the vitamin D derivatives of the present invention can be used as reagents for studying the

metabolism of active vitamin $D_3$ (i.e., 1α,25-dihydroxyvitamin $D_3$).

The vitamin D derivatives of Formula (1) and Formula (2) of the present invention are novel; although the method of synthesizing them is not limited at all, they may be synthesized, for example, according to the following method. The reference numerals designating compounds in the following synthesis method correspond to those in the reaction schemes of the later-described Examples, this is however, only for the purpose of clearly illustrating and making understandable the following method; the synthesis method of the present invention is not limited to the specific description of the later-described Examples.

Crystalline epoxide 1, obtainable from D-glucose and the like, can be used as a starting material. Namely, crystalline epoxide is reacted with an alkanediol, followed by the selective introduction of a functional group to the 3-position to give a 3-substituted derivative 2. Then, after protecting the hydroxy of the functional group at the 3-position to give compound 3, the benzylideneacetal moiety protecting the hydroxy at the 4- and 6-positions was brominated and cleaved by treatment with brominating reagent such as N-bromosuccinimide and the like, to give bromide 4. The thus obtained bromide 4 is converted to an alcohol 7 via a diol 5 and a tris-silyl ether 6. An olefin was formed at 5- and 6-positions of the alcohol by treatment with zinc powder to give a diol 8. By selective introduction of a leaving group to the primary hydroxy of the diol 8, a sulfonate 9 is obtained. Then, for example, via an epoxide 10, ethynyl is introduced to the 1-position to give an enyne 11. The enyne 11 is treated with potassium carbonate and the like to give an enyne 12. The hydroxy of the enyne 12 is silylated (e.g. tert-butyldimethylsilylated) to give a tris-silyl ether 13. By reacting the tris-silyl ether 13 with a desired bromoolefin 14 (CD-ring part) in an appropriate solvent using a palladium catalyst, a 2α-substituted D skeleton is constructed. The thus obtained protected derivative 15 is subjected to deprotection and purified by a usual manner such as reverse phase HPLC and thin layer chromatography to give the desired vitamin D derivative 16. Alternatively, the protected derivative 15 may be deprotected after purification.

As CD-ring part compounds of vitamin D derivatives, known compounds can be used. Alternatively, a desired CD-ring compound can be obtained by appropriately modifying a side chain of a known CD-ring compound. Alternatively, a CD-ring compound can be obtained from a known vitamin D derivative having a corresponding side chain.

## EXAMPLES

The present invention will be described specifically by way of the following Examples, which in no way limit the invention.

Reaction schemes carried out in Examples are shown below:

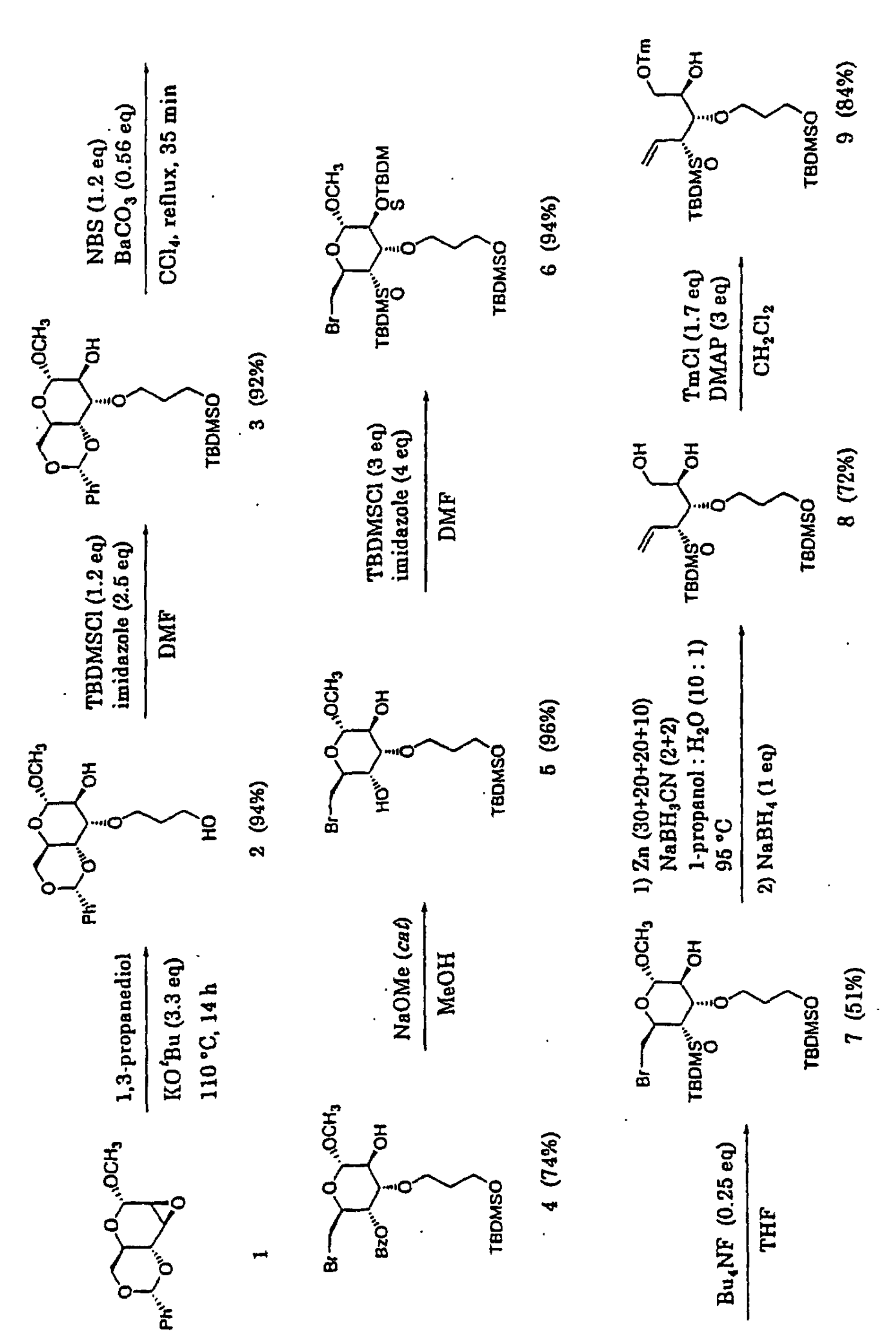
1
1,3-propanediol
KO$^t$Bu (3.3 eq)
110 °C, 14 h
2 (94%)
TBDMSCl (1.2 eq)
imidazole (2.5 eq)
DMF
3 (92%)
NBS (1.2 eq)
$BaCO_3$ (0.56 eq)
$CCl_4$, reflux, 35 min
4 (74%)
NaOMe (*cat*)
MeOH
5 (96%)
TBDMSCl (3 eq)
imidazole (4 eq)
DMF
6 (94%)
$Bu_4NF$ (0.25 eq)
THF
7 (51%)
1) Zn (30+20+20+10)
$NaBH_3CN$ (2+2)
1-propanol : $H_2O$ (10 : 1)
95 °C
2) $NaBH_4$ (1 eq)
8 (72%)
TmCl (1.7 eq)
DMAP (3 eq)
$CH_2Cl_2$
9 (84%)

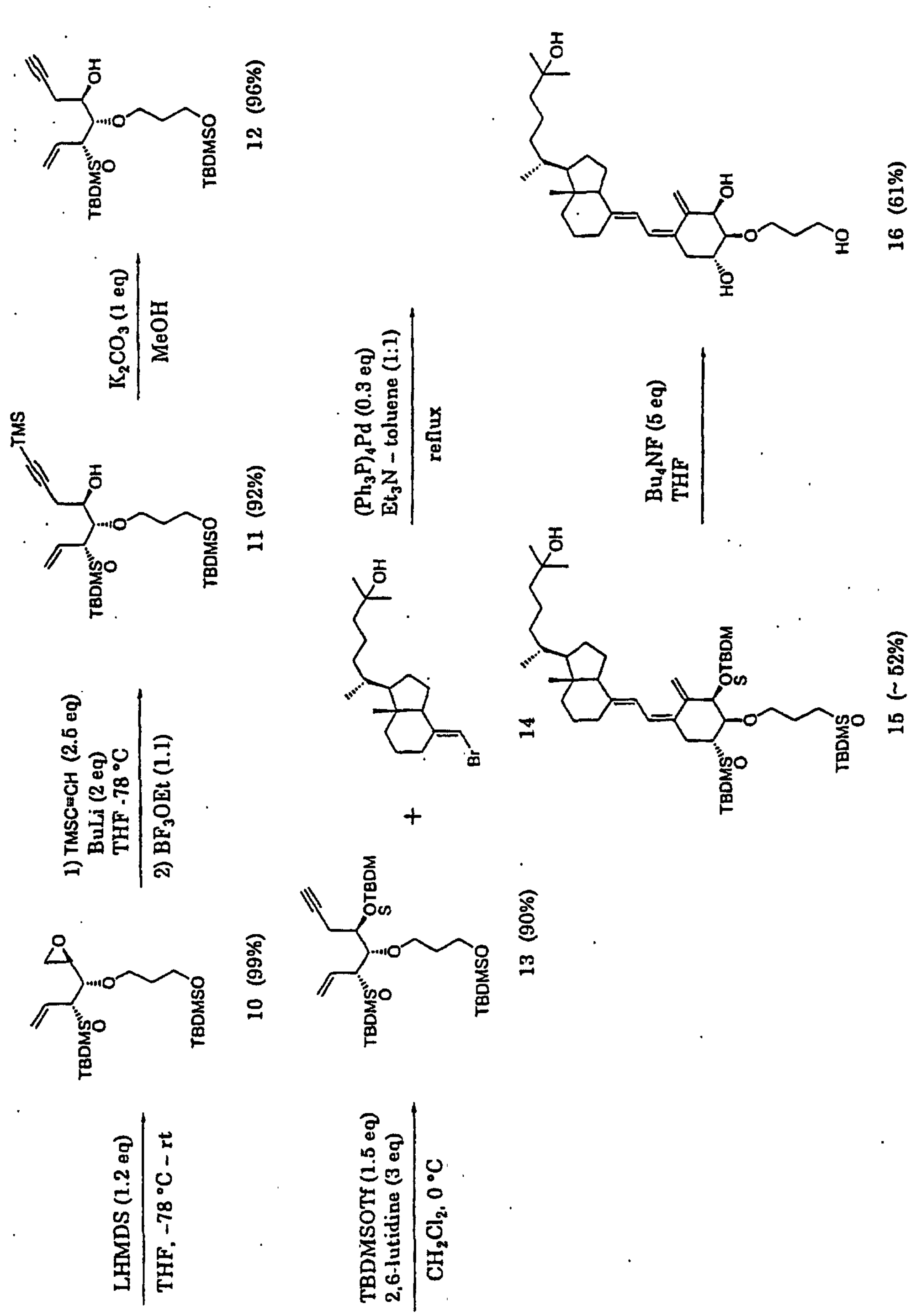


(Synthesis Example)

Synthesis of (5Z,7E)-(1S,2S,3R,20R)-2-(3-hydroxypropoxy)-9,10-seco-5,7,10(19)-cholestatriene-1,3,25-triol (Compound 16)

In the description of the following Examples, "%" means "volume %" unless otherwise mentioned.

(1) Synthesis of methyl-4,6-O-benzylidene-3-O-(3-hydroxypropyl)-α-D-altropyranoside (Compound 2)

Methyl 2,3-anhydro-4,6-O-benzylidene-α-D-mannopyranoside (Compound 1) (2.52 g, 9.54 mmol), an epoxide, was suspended in 1,3-propanediol (63 mL); potassium tert-butoxide (3.53 g. 31.5 mmol) was added to the suspension, which was heated at 110°C for 14 hours. The reaction mixture was partitioned between methylene chloride (400 mL) and saturated aqueous ammonium chloride (200 mL), and the aqueous layer was extracted with methylene chloride (200 mL) twice. The combined organic layers were dried over anhydrous sodium sulfate and purified by silica gel column chromatography (10%→66% ethyl acetate/hexane), to give diol Compound 2 (3.06 g, yield 94%) as a colorless oil.
$^1$HNMR(400MHz,$CDCl_3$) δ 1.75-1.91(2H,m), 2.42(1H,d.J=5.8Hz), 3.40(3H,s), 3.70-3.81(6H,m), 4.01-4.09(3H,m), 4.29-4.34(2H,m), 4.61(1H,s), 5.56(1H,s), 7.36-7.38(3H,m), 7.47-7.50(2H,m).
EIMS m/z 340($M^+$ )
HREIMS $C_{17}H_{24}O_7$ ($M^+$ ) calcd. 340.1522; found 340.1523

(2) Synthesis of methyl 4,6-O-benzylidene-3-O-[3-{(t-butyldimethylsilyl)oxy}propyl]-α-D-altropyranoside (Compound 3)

Compound 2 (3.06 g. 8.98 mmol) was dissolved in DMF (30 mL). tert-Butyldimethylsilyl chloride (1.62 g. 10.8 mmol) and imidazole (1.53 g. 22.5 mmol) were added to the resulting solution, which was stirred at room temperature for 3 hours. The reaction mixture was partitioned between ethyl acetate (500 mL) and water (150 mL). The organic layer was washed with water (150 mL) and saturated brine (150 mL) in that order, dried over anhydrous sodium sulfate and purified by silica gel column chromatography (10%→33% ethyl acetate/hexane) to give Compound 3 (3.74 g, yield 92%) as a colorless oil.
$^1$HNMR(400MHz,$CDCl_3$) δ 0.02 and 0.03(6H,each s), 0.87(9H,s), 1.78-1.82(2H,m), 1.93(1H,br), 3.39(3H,s), 3.65-3.80 (6H,m), 3.95(1H,dd,J=2.7 and 8.8Hz), 3.99-4.00(1H,m), 4.26-4.33(2H,m), 4.89(1H,s), 5.55(1H,s), 7.34-7.37(3H,m), 7.47-7.49(2H,m).
EIMS m/z 454($M^+$ ),423($M-OCH_3$)$^+$, 397(M- $^t$Bu)$^+$
HREIMS $C_{23}H_{38}O_7Si$($M^+$ ) calcd. 454.2387: found 454.2267

(3) Synthesis of methyl 4-O-benzoyl-6-bromo-3-O-[3-{(t-butyldimethylsilyl)oxy}propyl]-6-deoxy-α-D-altropyranoside (Compound 4)

Compound 3 (2.42 g. 5.31 mmol) was dissolved in carbon tetrachloride (53 mL). N-bromosuccinimide (1.04 g. 5.84 mmol) and barium carbonate (586.8 mg, 2.97 mmol) were added to the resulting solution, which was heated and refluxed for 40 min. After cooling the reaction mixture, ethyl acetate (50 mL) was added, and insoluble materials were removed through filter paper. The filtrate was partitioned between ethyl acetate (350 mL) and a 0.1N aqueous sodium thiosulfate solution (50 mL). The organic layer was washed with saturated sodium hydrogen carbonate (50 mL), water (50 mL) and saturated brine (50 mL) in that order, dried over anhydrous sodium sulfate and purified by silica gel column chromatography (10%→20% ethyl acetate/hexane) to give bromide Compound 4 (2.09 g, yield 74%) as a colorless oil.
$^1$HNMR(400MHz,$CDCl_3$) δ -0.04 and -0.03(6H, each s), 0.83(9H,s), 1.67-1.73(2H,m), 2.52(1H,br s), 3.50(3H,s), 3.55-3.70(6H,m), 3.73(1H,dd,J=4.0 and 7.3Hz), 3.97(1H,dd,J=3.3 and 7.3Hz), 4.35(1H,dt,J=3.7 and 7.0Hz), 4.70(1H, d,J=3.3Hz), 5.45(1H,dd,J=4.0 and 7.0Hz), 7.43-7.47(2H,m), 7.57-7.58(1H,m), 8.03-8.08(2H,m).
EIMS m/z 519 and 517(M-Me)$^+$, 503 and 501($M-OCH_3$)$^+$.
HREIMS $C_{23}H_{37}BrO_7Si$($M^+$ ) calcd. 532.1492; found 532.1489.

(4) Synthesis of methyl 6-bromo-3-O-[3-{(t-butyldimethylsilyl)oxy}propyl]-6-deoxy-α-D-altropyranoside (Compound 5)

Compound 4 (2.09 g. 3.92 mmol) was dissolved in $CH_3OH$ (50 mL). A 28% $NaOCH_3$ methanol solution (50 mL) was added to the resulting solution, and the reaction was carried out. at room temperature overnight. Silica gel (20 g) was added to the reaction mixture, which was then concentrated under reduced pressure. The thus obtained concentrated residue was purified by silica gel column chromatography (10%→33% ethyl acetate/hexane) to give diol Compound 5 (1.62 g, yield 96%) as a colorless oil.
$^1$HNMR(400MHz,$CDCl_3$) δ 0.06(6H,s), 0.89(9H,s), 1.73-1.85(2H,m), 2.05(1H,brs), 3.00(1H,d,J=8.8Hz), 3.43(3H,s), 3.54(1H,dd,J=7.3 and 10.6Hz), 3.59(1H,t,J=4.6Hz), 3.63(1H,ddd,J=5.5,7.3 and 9.2Hz), 3.69-3.82(5H,m), 3.93-3.98 (2H,m), 4.62(1H,d,J=2.2Hz).
FABMS m/z 469 and 467(M+K)$^+$, 453 and 451(M+Na)$^+$, 431 and 429(M+H)$^+$, 399 and 397($M-OCH_3$)$^+$, 73 and 371 (M- $^t$Bu)$^+$
HREIMS $C_{16}H_{33}BrO_6Si$($M^+$ ) calcd. 428.1230; found 428.1224.

(5) Synthesis of methyl 6-bromo-2,4-bis-(O-t-butyldimethylsilyl)-3-O-[3-{(t-butyldimethylsilyl)oxy}propyl]-6-deoxy-α-D-altropyranoside (Compound 6)

Compound 5 (1.84 g. 4.29 mmol) was dissolved in DMF (30 mL). Tert-butyldimethylsilyl chloride (1.94 g. 12.9 mmol) and imidazole (1.17 g. 17.2 mmol) were added to the resulting solution, which was stirred at room temperature overnight. The reaction mixture was partitioned between ethyl acetate (450 mL) and water (100 mL). The organic layer was washed with water (100 mL) and saturated brine (100 mL) in that order, dried over anhydrous sodium sulfate and purified by silica gel column chromatography (10% ethyl acetate/hexane) to give tris-silyl Compound 6 (2.66 g, yield 94%) as a colorless oil.
$^1$HNMR(400MHz,$CDCl_3$) δ 0.04,0.08,0.09 and 0.11(18H, each s), 0.89 and 0.90(27H, each s), 1.78-1.81(2H,m), 3.37 (3H,s), 3.40(1H,m), 3.48(1H,dd,J=7.3 and 10.6Hz), 3.55(1H,dt,J=6.2 and 8.8Hz), 3.64-3.72(4H,m), 3.92-3.96(2H,m), 4.12-4.16(1H,m), 4.48(1H,br s).
EIMS m/z 658 and 656($M^+$), 643 and 641$(M-Me)^+$, 601 and 599$(M-{}^tBu)^+$.

(6) Synthesis of methyl 6-bromo-4-(O-t-butyldimethylsilyl)-3-O-[3-{(t-butyldimethylsilyl)oxy}propyl]-6-deoxy-α-D-altropyranoside (Compound 7)

Compound 6 (2.78 g. 4.23 mmol) was dissolved in THF (25 mL). A 1M solution of tetrabutylammonium fluoride in THF (1.06 mL, 1.06 mmol) was added to the resulting solution, which was stirred at room temperature for 2.5 hours. Silica gel (10 g) was added to the reaction mixture, which was then concentrated under reduced pressure; the thus obtained concentrated residue was purified by silica gel column chromatography (1%→20% ethyl acetate/hexane) to give alcohol Compound 7 (1.16 g, yield 51%), as a colorless oil, and Compound 6 (647.1 mg, yield 23%), which was the starting material of this step.
$^1$HNMR(400MHz,$CDCl_3$) δ 0.05, 0.11 and 0.12(12H, each s), 0.89 and 0.90(18H, each s), 1.79(2H,ddd,J=2.6,6.2 and 12.8Hz), 2.06(1H,s), 3.42(3H,s), 3.48-3.50(1H,m), 3.52(1H,dd,J=6.2 and 10.8Hz), 3.61-3.68(3H,m), 3.69-3.73(2H,m), 3.93(1H,br), 3.96(1H,dd,J=3.3 and 8.1Hz), 4.11-4.15(1H,m), 4.59(1H,d,J=2.2Hz).
EIMS m/z 544 and 542($M^+$), 487 and 485$(M-{}^tBu)^+$

(7) Synthesis of (2R,3S,4R)-4-[(t-butyldimethylsilyl)oxy]-3-[3-{(t-butyldimethylsilyl)oxy}propoxy]hex-5-ene-1,2-diol (Compound 8)

Compound 7 (1.07 g. 1.97 mmol) was dissolved in 1-propanol (50 mL), and water (5 mL) was added to the solution. Zinc powder (3.86 g. 59.1 mmol) and sodium cyanoborohydride (247.9 mg, 3.94 mmol) were added to the resulting mixture, which was then stirred at 95°C for 20 min. After further addition of zinc power (2.58 g. 39.4 mmol), the reaction was carried out at the same temperature for 35 min., then zinc power (2.58 g. 39.4 mmol) and sodium cyanoborohydride (247.9 mg, 3.94 mmol) were further added, and the reaction was carried out at the same temperature for 30 min. Zinc powder (1.29 g. 19.7 mmol) was further added to the reaction mixture, which was then stirred for 45 min. at the same temperature, cooled and filtered for removing insoluble materials. Sodium borohydride (74.5 mg, 1.97 mmol) was added to the filtrate, which was then stirred for 10 min. at room temperature. Silica gel (1 g) was added, then the mixture was concentrated; the thus obtained concentrated residue was purified by silica gel column chromatography (2%→10% ethyl acetate/hexane) to give diol Compound 8 (615.7 mg, yield 72%) as a colorless oil.
$^1$HNMR(400MHz,$CDCl_3$) δ 0.04, 0.06 and 0.10(12H, each s), 0.89 and 0.90(18H, each s), 1.72-1.80(2H,m), 2.33(1H, t,J=6.0Hz), 3.20(1H,d,J=3.7Hz), 3.22(1H,dd,J=4.4 and 6.6Hz),3.54(1H,m), 3.62-3.75(4H,m), 3.79(1H,m), 3.86(1H,m), 4.36-4.38(1H,m), 5.20(1H,dt,J=1.5 and 10.6Hz), 5.29(1H,dt,J=1.5 and 17.2Hz), 5.89(1H,ddd,J=5.9,10.6 and 17.2Hz).
EIMS m/z 377$(M-{}^tBu)^+$

(8) Synthesis of (2R,3S,4R)-4-[(t-butyldimethylsilyl)oxy]-3-[3-{(t-butyldimethylsilyl)oxy}propoxy]-1-[(2,4,6-trimethylbenzenesulfonyl)oxy]hex-5-en-2-ol (Compound 9)

Compound 8 (482.5 mg, 1.11 mmol) was dissolved in $CH_2Cl_2$ (11 mL). Under cooling with ice, 2-mesitylenesulfony chloride (267.0 mg, 1.22 mmol) and DMAP (271.2 mg, 2.22 mmol) were added to the resulting solution, which was stirred at 0°C for 1 hour. 2-Mesitylenesulfony chloride (145.6 mg, 0.666 mmol) and DMAP (135.6 mg, 1.11 mmol) were further added to the reaction mixture, which was stirred at 0°C for 3 hours. The reaction mixture was partitioned between ethyl acetate (50 mL) and water (10 mL). The organic layer was washed with water (10 mL) and saturated brine (10 mL) in that order, dried over anhydrous sodium sulfate and purified by silica gel column chromatography (10%→17% ethyl acetate/hexane) to give sulfonate Compound 9 (573.1 mg, yield 84%) as a colorless oil.
$^1$HNMR (400MHz, $CDCl_3$) δ 0.03 and 0.07(12H, each s), 0.87 and 0.88(18H, each s), 1.69-1.76(2H,m), 2.62(9H,s), 3.18 (1H,dd,J=1.8 and 4.8Hz), 3.44(1H,dt,J=6.6 and 8.8Hz), 3.65(2H,t,J=6.0Hz), 3.74(1H,dt,J=6.4 and 9.2Hz), 3.90(1H,dd,J=8.2 and 11.9Hz), 4.01-4.05(2H,m), 4.38(1H,t,J=5.3Hz), 5.20(1H,d,J=10.6Hz), 5.29(1H,d,J=17.2Hz), 5.83 (1H,ddd,J=6.0,10.6 and 17.2Hz), 6.96 and 6.98 (2H, each s).
EIMS m/z 601$(M-Me)^+$, 559$(M-^tBu)^+$

(9) Synthesis of (3R,4S,5R)-3-[(t-butyldimethylsilyl)oxy]-4-[3-{(t-butyldimethylsilyl)oxy}propoxy]-5,6-epoxyhex-1-ene (Compound 10)

Compound 9 (306.7 mg, 0.497 mmol) was dissolved in THF (5 mL). A 1M solution of lithium hexamethyldisilazide in THF (0.596 mL, 0.596 mmol) was added to the resulting solution at -78°C, and then the temperature of the reaction mixture was gradually returned to room temperature. The reaction mixture was partitioned between ethyl acetate (200 mL) and saturated aqueous ammonium chloride (70 mL). The organic layer was washed with water (70 mL) and saturated brine (70 mL) in that order, dried over anhydrous sodium sulfate and purified by silica gel column chromatography (hexane→10% ethyl acetate/hexane) to give epoxide Compound 10 (204.7 mg, yield 99%) as a colorless oil.
$^1$HNMR(400MHz, $CDCl_3$) δ 0.04 and 0.08(12H, each s), 0.88 and 0.90(18H, each s), 1.77(2H, quintet J=6.2Hz), 2.58 (1H,dd,J=2.8 and 4.8Hz), 2.76(1H,t,J=4.8Hz), 2.82(1H,t,J=6.2Hz), 3.06(1H,ddd,J=2.8,4.8 and 6.2Hz), 3.55(1H,dt, J=6.2 and 9.5Hz), 3.69(2H,t,J=6.2Hz), 3.76(1H,dt,J=6.2 and 9.5Hz), 4.21(1H,brt,J=6.2Hz), 5.14(1H,d,J=10.3Hz), 5.28 (1H,d,J=17.2Hz),
5.88(1H,ddd,J=6.2,10.3 and 17.2Hz). EIMS m/z 359 $(M-^tBu)^+$

(10) Synthesis of (4R,5S,6R)-6-[(t-butyldimethylsilyl)oxy]-5-[3-{(t-butyldimethylsilyl)oxy}propoxy]-1-(trimethylsilyl)oct-7-en-1-yn-4-ol (Compound 11)

Compound 10 (155.9 mg, 0.374 mmol) was dissolved in THF (5 mL). Meanwhile, trimethylsilylacetylene (0.132 mL, 0.935 mmol) was dissolved in THF (5 mL) and mixed with a butyl lithium hexane solution (1.61M, 0.465 mL, 0.748 mmol) at -78°C and stirred for 5 min. To the solution, the solution of Compound 10 in THF was added at -78°C, boron trifuoride diethy etherate (52.1 µl, 0.411 mmol) was further added, and the reaction temperature was gradually raised to 10°C. The reaction mixture was partitioned between ethyl acetate (50 mL) and saturated aqueous ammonium chloride (10 mL). The organic layer was washed with saturated aqueous sodium hydrogen carbonate (10 mL), water (10 mL) and saturated brine (10 mL) in that order, dried over anhydrous $Na_2SO_4$ and purified by silica gel column chromatography (hexane→8% ethyl acetate/hexane) to give olefin Compound 11 (178.0 mg, yield 92%) as a colorless oil.
$^1$HNMR(400MHz,$CDCl_3$) δ 0.04, 0.05, 0.10 and 0.13(15H, each s), 0.89 and 0.91(18H, each s), 1.78-1.81(2H,m), 2.47 (1H,dd,J=5.9 and 16.9Hz), 2.53(1H,dd,J=8.4 and 16.9Hz), 3.24(1H,d,J=4.4Hz), 3.31(1H,dd,J=2.2 and 4.4Hz), 3.58 (1H,dt,J=6.6 and 9.2Hz), 3.70(2H,t,J=5.9Hz), 3.81(1H,dt,J=6.2 and 9.2Hz), 3.92-3.98(1H,m), 4.42(1H,dd,J=4.4 and 5.9Hz), 5.20(1H,d,J=10.6Hz), 5.31(1H,d,J=17.2Hz), 5.89(1H,ddd,J=5.9,10.6 and 17.2Hz)
EIMS m/z: 499$(M-Me)^+$, 457$(M-^tBu)^+$

(11) Synthesis of (4R,5S,6R)-6-[(t-butyldimethylsilyl)oxy]-5-[3-{(t-butyldimethylsilyl)oxy}propoxy]oct-7-en-1-yn-4-ol (Compound 12)

Compound 11 (233.6 mg, 0.454 mmol) was dissolved in methanol (10 mL). Potassium carbonate (62.7 mg, 0.454 mmol) was added to the resulting solution, which was stirred at room temperature for 6 hours. After adding acetic acid (52.0 mL, 0.908 mmol), the reaction mixture was concentrated; the residue was partitioned between ethyl acetate (100 mL) and water (30 mL). The organic layer was washed with water (30 mL) and saturated brine (30 mL) in that order, dried over anhydrous sodium sulfate and purified by silica gel column chromatography (hexane→4% ethyl acetate/hexane) to give enyne Compound 12 (193.6 mg, yield 96%) as a colorless oil.
$^1$HNMR(400MHz,$CDCl_3$) δ 0.05, 0.06 and 0.11(12H, each s), 0.89 and 0.91(18H, each s), 1.76-1.83(2H,m), 1.99(1H, t,J=2.7Hz), 2.48(2H,m), 3.27(1H,dd,J=2.2 and 4.4Hz), 3.33(1H,d,J=4.4Hz), 3.58(1H,dt,J=6.6 and 9.2Hz), 3.68-3.73 (2H,m), 3.80(1H,dt,J=6.2 and 9.2Hz), 3.96-4.02(1H,m), 4.43(1H,ddt,J=1.5,4.4 and 5.9Hz), 5.22(1H,dt,J=1.5 and 10.6Hz), 5.32(1H,dt,J=1.5 and 17.2Hz), 5.89(1H,ddd,J=5.9,10.6 and 17.2Hz)
EIMS m/z: 385$(M-^tBu)^+$

(12) Synthesis of (3R,4S,5R)-3,5-bis-[(t-butyldimethylsilyl)oxy]-4-[3-{(t-butyldimethylsilyl)oxy}propoxy]oct-1-en-7-yne (Compound 13)

Compound 12 (193.6 mg, 0.437 mmol) was dissolved in $CH_2Cl_2$ (5 mL). Under cooling with ice, tert-butyldimethylsilyltriflate (0.151 mL, 0.656 mmol) and 2,6-lutidine (0.153 mL, 1.31 mmol) were added to the resulting solution, which was stirred at 0°C for 1 hour. The reaction mixture was partitioned between ethyl acetate (150 mL) and saturated

aqueous sodium hydrogen carbonate (30 mL). The organic layer was washed with water (30 mL) and saturated brine (30 mL) in that order and purified by silica gel column chromatography (hexane→3% ethyl acetate/hexane) to give tris-silyl Compound 13 (219.3 mg, yield 90%) as a colorless oil.
$^1$HNMR(400MHz,$CDCl_3$) δ 0.03, 0.05, 0.07, 0.09 and 0.10(18H, each s), 0.89 and 0.90(27H, each s), 1.74-1.80(2H, m), 1.95(1H,t,J=2.6Hz), 2.35(1H,ddd,J=2.6,5.5 and 16.9Hz), 2.49(1H,ddd,2.6,5.5 and 16.9Hz), 3.35(1H,dd,J=3.5 and 5.5Hz), 3.60-3.76(4H,m), 3.88(1H,q,J=5.5Hz), 4.30(1H,dd,J=3.5 and 7.0Hz), 5.12(1H,br d,J=10.3Hz), 5.20(1H,dt, J=1.3 and 17.2Hz), 5.95(1H,ddd,J=7.0,10.3 and 17.2Hz)
EIMS m/z: 541$(M-Me)^+$, 499$(M-{}^tBu)^+$

(13) Synthesis of Compound 16 by the condensation of Compound 13 and Compound 14, followed by deprotection

Compound 13 (219.3 mg, 0.394 mmol) and Compound 14 (129.6 mg, 0.363 mmol), which was a vinyl bromide, were dissolved in toluene (5 mL) and mixed with triethylamine (5 mL). Tetrakis(triphenylphosphine)palladium (125.8 mg, 0.109 mmol) was added to the resulting solution, which was then refluxed at 120°C for 90 min. Ethyl acetate (10 mL) was added to the reaction mixture, which was then subjected to a silica gel pad to filter off insoluble materials. The filtrate was concentrated under reduced pressure using a rotary evaporator and roughly purified by silica gel column chromatography (hexane→6% ethyl acetate/hexane) to give 1,3-bis-(O-t-butyldimethylsilyl)-2a-[3-{(t-butyldimethylsilyl)oxy}propoxy]-1α,25-dihydroxyvitamin $D_3$ (Compound 15) as a condensation product.
Compound 15 (156.6 mg) was dissolved in THF (3 mL). A solution of tetrabutylammonium fluoride in THF (1M, 0.940 mL, 0.940 mmol) was added to the resulting solution, and reaction was carried out for 96 hours at room temperature. The reaction mixture was concentrated under reduced pressure by a rotary evaporator. The concentrated residue was dissolved in methanol (1 mL) and adsorbed to a silica gel TLC plate (20 × 20 cm, 0.5 mm thick; 10% MeOH in $CH_2Cl_2$ ) for purification, giving approximately 75.0 mg of Compound 16 which was a vitamin D derivative. Repeated purification using a silica gel TLC plate gave 56.3 mg of Compound 16 (yield 32%). Purification of Compound 16 using reverse phase HPLC (purification conditions: reverse phase HPLC YMC-Pack ODS column, 20 x 150 mm, 9.0 mL/min, $CH_3CN$:water=3:2) gave white powder.
Data of Compound 16 purified by reverse phase HPLC $^1$HNMR(400MHz,$CDCl_3$) δ 0.54(3H,s), 0.93(3H,d,J=6.4Hz), 1.21(6H,s), 2.24(1H,dd,J=9.2 and 13.4Hz), 2.69(1H,dd,J=4.4 and 13.4Hz), 2.81-2.83(1H,m), 3.38(1H,dd,J=3.2 and 7.5Hz),4.03-4.08(1H,m), 4.44(1H,brd,J=2.8Hz), 5.10(1H,d,J=1.8Hz), 5.39(1H,br s), 6.01(1H,d,J=11.3Hz), 6.42(1H,d, J=11.3Hz)
EIMS m/z: 490$(M^+)$, 472$(M-H_2O)^+$, 454$(M-2H_2O)^+$
HREIMS $C_{30}H_{50}O_5$ $(M^+)$ calcd. 490.3660; found 490.3638

(Test example) Assay for binding to bovine thymus vitamin D receptor (VDR)

Binding property to bovine thymus vitamin D receptor was tested for the vitamin D derivative of the present invention obtained according to the above Synthesis Example.
Ethanol solution of 1α,25-dihydroxyvitamin $D_3$ (the standard substance) and that of the vitamin D derivative of the present invention were prepared at various concentrations. Bovine thymus 1α,25-dihydroxyvitamin $D_3$ receptor was purchased from Yamasa Biochemcal (Choshi, Chiba, Japan) (lot.110431) and one ampule (approximately 25 mg) of the receptor was dissolved in 55 mL of 0.05 M phosphate 0.5M potassium buffer (pH 7.4) just before use.
Each of the ethanol solutions (50 µl) of vitamin D derivative of the present invention and 1α,25-dihydroxyvitamin $D_3$ was put into a respective tube with 500 µl (0.23 mg protein) of the receptor solution, preincubated at room temperature for 1 hour, and $[^3H]$-1α,25-dihydroxyvitamin $D_3$ was added at the final concentration of 0.1 nM, followed by incubation overnight at 4°C. Each of the reaction mixtures was mixed with DCC (dextran coated charcoal), left for 30 minutes at 4°C and centrifuged at 3000 rpm for ten minutes to separate the bound and free forms of $[^3H]$-1α, 25-dihydroxyvitamin $D_3$. Each of the resultant supernatants (500 µl) was mixed with ACS-II (9.5 ml) (AMERSHAM, England) for radioactivity measurement.
The binding property of the vitamin D derivative of the present invention was 180 when expressed in relative value with that of 1α,25-dihydroxyvitamin $D_3$ taken as 100. The value was calculated according to the following equation:

$$X=(y/x) \times 100$$

X: relative VDR binding property of the vitamin D derivative of the present invention
y: concentration of 1α,25-dihydroxyvitamin $D_3$ that inhibits 50% of the binding of $[^3H]$1α,25-dihydroxyvitamin $D_3$ and VDR

x: concentration of the vitamin D derivative of the present invention that inhibits 50% of the binding of [$^3$H]1α, 25-dihydroxyvitamin $D_3$ and VDR

INDUSTRIAL APPLICABILITY

Vitamin D derivatives represented by Formulae (I) and (II) of the present invention are novel compounds and exhibit excellent physiological activity; they may be useful as medicines for diseases, such as renal bone diseases, hypoparathyroidism, osteoporosis, etc.

## Claims

**1.** A vitamin D derivative of Formula (1):

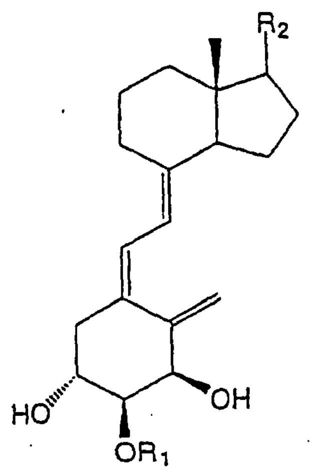

wherein
$R_1$ and $R_2$ are straight chained or branched lower alkyl which may be substituted with hydroxy.

**2.** The vitamin D derivative of claim 1, wherein $R_1$ is straight chained $C_{2-6}$ alkyl substituted with hydroxy and $R_2$ is straight chained or branched $C_{1-12}$ alkyl substituted with hydroxy.

**3.** The vitamin D derivative of claim 1, wherein $R_1$ is straight chained $C_{2-4}$ alkyl substituted with hydroxy and $R_2$ is straight chained or branched $C_{3-10}$ alkyl substituted with hydroxy.

**4.** A vitamin D derivative of Formula (2):

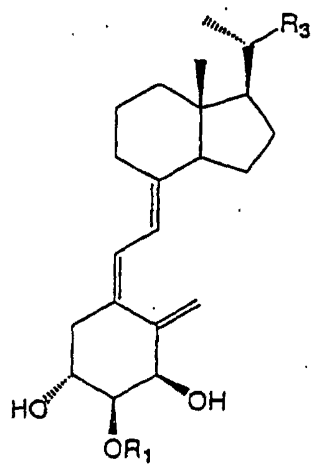

wherein
$R_1$ is 2-hydroxyethyl or 3-hydroxypropyl and $R_3$ is 4-hydroxy-4-methylpentyl or 4-ethyl-4-hydroxyhexyl.

**5.** The vitamin D derivative of claim 4, wherein $R_1$ is 3-hydroxypropyl and $R_3$ is 4-hydroxy-4-methylpentyl.

# INTERNATIONAL SEARCH REPORT

International application No.
PCT/JP01/01451

**A. CLASSIFICATION OF SUBJECT MATTER**
Int.Cl$^7$ C07C401/00 // A61K31/59, A61P3/02

According to International Patent Classification (IPC) or to both national classification and IPC

**B. FIELDS SEARCHED**

Minimum documentation searched (classification system followed by classification symbols)
Int.Cl$^7$ C07C401/00, A61K31/59, A61P3/02

Documentation searched other than minimum documentation to the extent that such documents are included in the fields searched

Electronic data base consulted during the international search (name of data base and, where practicable, search terms used)
CAPLUS (STN), REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| PX | KITTAKA, Atsushi et al., "A Concise and Efficient Route to 2α-(ω-Hydroxyalkoxy)- 1α, 25-dihydroxyvitamin D3: Remarkably High Affinity to Vitamin D Receptor", Org. Lett., 2000, Vol.2, No.17, pages 2619 to 2622 | 1~5 |
| A | EP, 806413, A1 (Chugai Seiyaku Kabushiki Kaisha), 12 November, 1997 (12.11.97), & JP, 8-259526, A & WO, 96/22973, A1 | 1~5 |
| A | JP, 63-107929, A (Chugai Pharmaceutical Co., Ltd.), 12 May, 1988 (12.05.88) (Family: none) | 1~5 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 30 May, 2001 (30.05.01) | 12 June, 2001 (12.06.01) |
| Name and mailing address of the ISA/ Japanese Patent Office | Authorized officer |
| Facsimile No. | Telephone No. |

Form PCT/ISA/210 (second sheet) (July 1992)